**Europäisches Patentamt**

**European Patent Office**

**Office européen des brevets**

(11) Veröffentlichungsnummer: **0 181 999**
**A1**

## (12) EUROPÄISCHE PATENTANMELDUNG

(21) Anmeldenummer: **85107496.3**

(22) Anmeldetag: **18.06.85**

(51) Int. Cl.⁴: **B 01 J 23/78**
// (C07B35/04,
C07C5:333, 15:46)

(30) Priorität: **22.11.84 DE 3442636**

(43) Veröffentlichungstag der Anmeldung: **28.05.86**
**Patentblatt 86/22**

(84) Benannte Vertragsstaaten: **BE DE FR GB IT NL**

(71) Anmelder: **SÜD-CHEMIE AG, Lenbachplatz 6,**
**D-8000 München 2 (DE)**

(72) Erfinder: **Kremer, Hans-Joachim, Dr., Zugspitzstrasse 7,**
**D-8011 Poing (DE)**
Erfinder: **Dethy, Jacques Maurice, Dr., Avenue de l'oud**
**Kabelleke 10, B-1140 Brüssel (BE)**

(74) Vertreter: **Patentanwälte Dipl.-Ing. Splanemann**
**Dipl.-Chem. Dr. B. Reitzner, Tal 13,**
**D-8000 München 2 (DE)**

(54) **Dehydrierungskatalysator.**

(57) Ein Dehydrierungskatalysator auf der Basis von $Fe_2O_3$ und $K_2O$ kann dadurch erhalten werden, daß man ein wasserhaltiges Gemisch aus

(a) 45 bis 90 Gew.-% $Fe_2O_3$ und/oder mindestens einer in $Fe_2O_3$ überführbaren Eisenverbindung (letztere berechnet als $Fe_2O_3$),

(b) 5 bis 40 Gew.-% $K_2O$ und/oder mindestens einer in $K_2O$ überführbaren Kaliumverbindung (letztere berechnet als $K_2O$,

(c) 3 bis 30 Gew.-% MgO und/oder mindestens einer in MgO überführbaren Magnesiumverbindung (letztere berechnet als MgO),

(d) 0 bis 10 Gew.-% einer Chrom- und/oder einer Manganverbindung, berechnet als $Me_2O_3$,

(e) 0 bis 10 Gew.-% einer Verbindung des Cers, Molybdäns, Wolframs oder deren Gemische, berechnet als das unter Normbedingungen stabilste Oxid und

(f) 0 bis 15 Gew.-% und/oder mindestens einer CaO enthaltenden oder in CaO überführbaren Verbindung (letztere berechnet als CaO)

knetet und zu Formlingen verformt, die Formlinge trocknet und die getrockneten Formlinge bei Temperaturen von 500 bis 750 °C calciniert.

Der Katalysator wird zur Dehydrierung von Kohlenwasserstoffen, insbesondere von Ethylbenzol, in Gegenwart von Wasserdampf, verwendet.

ACTORUM AG

Die Erfindung betrifft einen Dehydrierungskatalysator und dessen Verwendung zur Dehydrierung von Kohlenwasserstoffen.

Die katalytische Dehydrierung von Alkylaromaten, aber auch von Monoolefinen, in Gegenwart von Wasserdampf im Temperaturbereich von 500 bis 700°C ist ein technisch in großem Umfang ausgeübtes Verfahren. Eine allgemeine Beschreibung dieses Verfahrens findet sich im "Kunststoff-Handbuch", Band V, Polystyrol, Karl-Hauser-Verlag, 1969, Seite 39 bis 47. Als Dehydrierungskatalysatoren werden Katalysatoren auf der Basis von $Fe_2O_3$ und $K_2O$ verwendet. Die für dieses Verfahren wirkungsvollsten Katalysatoren bestehen im wesentlichen aus $Fe_2O_3$, $K_2O$ und Chromoxid als Stabilisator. Derartige Katalysatoren sind beispielsweise in der US-PS 29 90 432 beschrieben. Diese Katalysatoren enthalten als hydraulisches Bindemittel Portland-Zement, der seinerseits einen gewissen Anteil an Magnesiumoxid enthält. Die Verwendung von hydraulischen Bindemitteln hat keinen Einfluß auf die Heißdampfstabilität der Katalysatoren.

Entsprechende Katalysatoren auf der Basis von $Fe_2O_3$, $K_2O$ und Chromoxid sind auch aus der PCT-Anmeldung US/82/01120 (WO 83/00687) bekannt.

Die heute gebräuchlichen Dehydrierungskatalysatoren enthalten neben $Fe_2O_3$, $K_2O$ und $Cr_2O_3$ zusätzlich Selektivitätspromotoren in Form von Oxiden der Elemente der 5. und 6. Nebengruppe des Periodensystems und der seltenen Erden. Derartige Selektivitätspromotoren sind beispielsweise die Oxide des Vanadiums (US-PS 33 61 683 und US-PS 30 84 125) oder insbesondere ein Gemisch der Oxide des Cers und Molybdäns (US-PS 39 04 552) bzw. des Cers und des Vanadiums (DE-OS 28 15 812).

Die Dehydrierungskatalysatoren nach der DE-OS 28 15 812 können auch kleinere Mengen an Magnesiumoxid enthalten. Die Verwendung von Vanadiumoxid ist für diese Katalysatoren zwingend vorgeschrieben. Außerdem müssen diese Katalysatoren

- 2 -

bei Temperaturen von mehr als 800°C calciniert werden, damit sie nicht zerfallen, wenn sie mit kondensierendem Heißdampf in Berührung kommen.

Dies gilt auch für die Katalysatoren nach den zuerst genannten Druckschriften. Die Herstellung aller dieser Katalysatoren ist daher energieaufwendig.

Mit der Verbesserung der Dehydriertechnologie, insbesondere der Styrolanlagentechnologie, die insbesondere durch den Betrieb im Vakuum gekennzeichnet ist, sind auch die Anforderungen an die Katalysatoren gestiegen, und dies nicht nur im Bezug auf die Aktivität und Selektivität, sondern vor allem auch in Bezug auf die physikalische Integrität, da die Gefahr einer Kondensation des Heißdampfes bei der Inbetriebnahme im Vakuum wesentlich größer ist als unter Druckbetrieb. So ist ein Katalysator, der bereits in frischem Zustand im Siedewasser zerfällt, für diese neue Technologie nicht geeignet. Die Beständigkeit gegenüber Siedewasser kann zwar durch sehr hohe Calciniertemperaturen im Bereich von 800 bis 1000°C erreicht werden. Abgesehen davon, daß diese hohen Calciniertemperaturen energieaufwendig sind, wurde aber festgestellt, daß bei diesen Temperaturen die Aktivität der Katalysatoren, insbesondere der selektiven Katalysatoren erheblich herabgesetzt wird.

Der Erfindung liegt somit die Aufgabe zugrunde, einen Dehydrierungskatalysator vorzuschlagen, der selbst bei niedriger Calcinierungstemperatur in kochendem Wasser nicht zerfällt und insbesondere im Anwendungsbereich niedriger Dampf/Kohlenwasserstoff-Molverhältnisse von 0,6 bis 8,8 den bisherigen Dehydrierungskatalysatoren bezüglich Aktivität und Selektivität mindestens äquivalent ist.

Es wurde überraschenderweise gefunden, daß Zusätze von Magnesium, die Widerstandsfähigkeit eines frischen Katalysators gegen Siedewasser derart verbessern, daß Calciniertemperaturen im Bereich von 500 bis 750°C zu seiner Herstellung völlig ausreichen.

Gegenstand der Erfindung ist somit ein Dehydrierungskatalysator auf der Basis von $Fe_2O_3$ und $K_2O$, der dadurch erhältlich ist, daß man ein wasserhaltiges Gemisch aus

(a) 45 bis 90 Gew.-% $Fe_2O_3$ und/oder mindestens einer in $Fe_2O_3$ überführbaren Eisenverbindung (letztere berechnet als $Fe_2O_3$),

(b) 5 bis 40 Gew.-% $K_2O$ und/oder mindestens einer in $K_2O$ überführbaren Kaliumverbindung (letztere berechnet als $K_2O$,

(c) 4 bis 30 Gew.-% MgO und/oder mindestens einer in MgO überführbaren Magnesiumverbindung (letztere berechnet als MgO),

(d) 0 bis 10 Gew.-% einer Chrom- und/oder einer Manganverbindung, berechnet als $Me_2O_3$

(e) 0 bis 10 Gew.-% einer Verbindung des Cers, Molybdäns, Wolframs oder deren Gemische, berechnet als das unter Normbedingungen stabilste Oxid,

(f) 0 bis 15 Gew.-% CaO und/oder mindestens einer CaO enthaltenden oder in CaO überführbaren Verbindung (letztere berechnet als CaO)

knetet und zu Formlingen verformt, die Formlinge trocknet und die getrockneten Formlinge bei Temperaturen von 500 bis 750°C calciniert.

- 4 -

Zu den erfindungsgemäß verwendbaren Eisenverbindungen, die in $Fe_2O_3$ überführbar sind, zählen z.B. die verschiedenen Ferri-Oxidhydrate, Eisen-(III)-Nitrat, Eisen-(III)-Oxalat usw. Auch zweiwertige Eisenverbindungen, wie Eisen-(II)-Sulfat können unter oxidierenden Bedingungen in $Fe_2O_3$ übergeführt werden.

Zu den Kaliumverbindungen, die in $K_2O$ überführbar sind, zählen beispielsweise Kaliumcarbonat, Kaliumbicarbonat, Kaliumhydroxid, Kaliumoxalat usw.

Zu den Magnesiumverbindungen, die in $MgO$ überführbar sind, zählen beispielsweise Magnesiumhydroxid, -carbonat, -acetat, -phosphat, -borat, -nitrat, -sulfat usw.

Anstelle von Gemischen von $Fe_2O_3$ und $MgO$ können auch die entsprechenden komplexen Oxide, z.B. die Spinelle und Ferritte, verwendet werden.

Die Verbindungen, die als Komponente (d) verwendet werden, dienen als Stabilisatoren. Vorzugsweise verwendet man die entsprechenden Oxide bzw. Verbindungen, die in die entsprechenden Oxide umwandelbar sind, z.B. die Hydroxide, Carbonate, Nitrate usw.

Die Chromverbindung kann auch als Kaliumchromat bzw. Kaliumbichromat vorliegen, wobei das Kalium dann einen Teil der Komponente (b) bildet. In entsprechender Weise kann als Manganverbindung beispielsweise Kaliumpermanganat verwendet werden.

Die Verbindungen der Komponente (e) dienen als Selektivitätspromotoren. Es können jeweils die Oxide eingesetzt werden bzw. Verbindungen, die in die Oxide überführbar sind. Die bei Normbedingungen (Luftatmosphäre bei einem Druck von 1 bar und einer Temperatur von 25°C) stabilsten Oxide sind $Ce_2O_3$, $MoO_3$ und $WO_3$. Es können auch komplexe Oxide, z.B.

Kaliummolybdat oder Kaliumwolframat verwendet werden.

Vorzugsweise enthält der erfindungsgemäße Katalysator
50 bis 65 Gew.-%, insbesondere    55 bis    65 Gew.-%
der Komponente (a)
5 bis 35 Gew.-%, insbesondere    10 bis    30 Gew.-%
der Komponente (b)
5 bis 20 Gew.-%, insbesondere    6 bis    15 Gew.-%
der Komponente (c),
0 bis 5 Gew.-%, insbesondere    0 bis    3 Gew.-%
der Komponente (d) und
0 bis 6 Gew.-%, insbesondere    0 bis    4 Gew.-%
der Komponente (e).
0 bis 10 Gew.-%, insbesondere    0 bis    5 Gew.-%
der Komponente (f).

Änderungen in der Zusammensetzung des Katalysators hängen
zum Teil von der Prozeßökonomie der Betriebsanlage ab.

Ein Katalysator im Anwendungsbereich von Dampf/Kohlenwasserstoff-Molverhältnissen unter 7 enthält zweckmäßig
50 bis 65 Gew.-% der Komponente (a), 15 bis 30 Gew.-%
der Komponente (b), bis zu 15 Gew.-%, vorzugsweise bis zu
10 Gew.-% der Komponente (c) und bis zu 3 Gew.-%, vorzugsweise bis zu 2 Gew.-% der Komponente (d).

Ein Katalysator im Anwendungsbereich von Dampf/Kohlenwasserstoff/Molverhältnissen über 5 enthält vorzugsweise
50 bis 65 Gew.-% der Komponente (a), 15 bis 25 Gew.-% der
Komponente (b), bis zu 10 Gew.-% der Komponente (c),
0 bis 3 Gew.-% der Komponente (d) und 0,01 bis 6, vorzugsweise 0,1 bis 5, insbesondere 0,1 bis 3 Gew.-% der Komponente (e).

Die Herstellung der erfindungsgemäßen Katalysatoren erfolgt
in der Regel durch Mischen der Komponenten (a) bis (c)
und gegebenenfalls der Komponente(n) (d), (e) und/oder (f).
Die Komponenten können trocken vorgemischt werden. Bevorzugt
werden sie jedoch unter Zusatz von Wasser, gegebenenfalls auch unter Zusatz von die Porosität verbessernden Mitteln, wie Stärke oder Methylcellulose und/oder unter Zusatz von Verformungshilfsmitteln, wie Graphit, miteinander vermischt.

Weiterhin können hydraulische Bindemittel, wie Portland-Zement oder Calciumaluminat-Zement zugesetzt werden. Durch das hydraulische Bindemittel wird die Festigkeit des trockenen Katalysators verbessert; es hat aber keinen Einfluß auf die Heißdampfstabilität. Das hydraulische Bindemittel ist in derRegel die Quelle für die Komponente (f).

Falls zunächst eine trockne Vormischung hergestellt wurde, wird diese mit Wasser versetzt und wie die Mischung, der schon zu Beginn Wasser zugesetzt wurde, geknetet und verformt. Vorzugsweise wird die Mischung extrudiert, obwohl auch Pillen, Kugeln, Ringe oder andere Formkörper wie z.B.gerippte Strangpreßlinge, gerippte Ringe oder Kleeblattstrukturen hergestellt werden können. Die so erhaltenen Extrudate oder die anderen Formkörper werden getrocknet, wobei ein Temperaturbereich von etwa 60 bis 300°C eingehalten wird. Anschließend werden die getrockneten Formkörper bei Temperaturen im Bereich von 500 bis 750°C calciniert.

Der so erhaltene Katalysator hat vorzugsweise eine BET-Oberfläche von weniger als 10m²/g, insbesondere von weniger als 5 m²/g.

Gegenstand der vorliegenden Erfindung ist ferner die Verwendung der vorstehend definierten Katalysatoren zur Dehydrierung von Kohlenwasserstoffen, insbesondere von Ethylbenzol, in Gegenwart von Wasserdampf. Das bei der Dehydrierung erhaltene Styrol wird auf Polystyrol bzw. auf Styrol-Mischpolymerisate verarbeitet.

Weitere Kohlenwasserstoffe, die mit Hilfe des erfindungsgemäßen Katalysators dehydriert werden können, sind beispielsweise Olefine oder alkylierte Benzole wie Dimethylbenzol, Isopropylbenzol u.dgl.

Die Erfindung ist durch die nachstehenden Beispiele erläutert.

### Beispiel 1

(Vergleichskatalysator)

55,9 g Eisenoxidpigment ($Fe_2O_3$) werden mit 3,6 g $Cr_2O_3$ trocken vermischt. Das Pulvergemisch wird mit einer wäßrigen Kaliumcarbonatlösung versetzt ($K_2CO_3$-Gehalt = 59.4 g), so daß eine knetbare Masse entsteht. Diese wird einige Minuten geknetet und dann zu Strangpreßlingen mit einem Durchmesser von etwa 3 mm und einer Länge von etwa 6 mm extrudiert. Die Trocknung erfolgt stufenweise bei 60°, 120° und 300°C über jeweils 2 Stunden; die getrockneten Strangpreßlinge werden in Luft 4 Stunden bei 540°C calciniert.

### Beispiel 2

Die Arbeitsweise von Beispiel 1 wird mit der Abweichung wiederholt, daß 63,3 g $Fe_2O_3$, 9,1 g MgO und 1,7 g $Cr_2O_3$ mit einer Kaliumhydroxidlösung (30,8 g KOH) angeteigt werden. Die weitere Behandlung erfolgt wie nach Beispiel 1.

### Beispiel 3

(Vergleichskatalysator)

Die Arbeitsweise von Beispiel 1 wird mit der Abweichung wiederholt, daß 58,1 g $Fe_2O_3$, 6 g $CeO_2$, 2,8 g $MoO_3$ und 8 g Portland-Zement mit einer Kaliumcarbonatlösung (36,8 g $K_2CO_3$) angeteigt und anschließend in der gleichen Weise wie nach Beispiel 1 behandelt werden. Wie in Tabelle I angegeben ist, zerfielen die Strangpreßlinge trotz Zementzusatz im Heißwassertest.

- 8 -

Beispiel 4
_____

Die Arbeitsweise von Beispiel 1 wird mit der Abweichung
wiederholt, daß 144 g $Fe_2(SO_4)_3$ zunächst thermisch in $Fe_2O_3$
übergeführt werden. Dieses wird mit 14,4 g $MgCO_3$, 6,0 g
$CeO_2$ und 3,0 g $MoO_3$ vermischt und mit einer wäßrigen Kaliumcarbonatlösung (39 g $K_2CO_3$) angeteigt. Die weitere Behandlung
erfolgt wie in Beispiel 1 angegeben (Calciniertemperatur
510°C).

Beispiel 5
_____

(Vergleichskatalysator)

Die Arbeitsweise von Beispiel 2 wird mit der Abweichung
wiederholt, daß die Mengen der Komponenten, wie in Tabelle
I angegeben, variiert werden. Die Calciniertemperatur beträgt
530°C.

Beispiel 6
_____

Die Arbeitsweise von Beispiel 3 wird mit der Abweichung
wiederholt, daß statt $Fe_2O_3$ und Magnesiumcarbonat ein vorgeformter Spinell ($MgFe_2O_4$) verwendet wird, wobei die in
Tabelle I angegebenen Mengenverhältnisse verwendet werden.
Die Calciniertemperatur beträgt 550°C.

Die nach den Beispielen 1 bis 6 hergestellten Katalysatoren
wurden dem Siedewassertest (HWT-Test) unterzogen, der wie
folgt durchgeführt wurde:

Destilliertes Wasser wird in einem 100ml-Glasbecher zum Sieden
gebracht. Anschließend werden 15 Pellets Dehydrierungskatalysator zugegeben, und nach 10 Minuten werden die vollständig
erhaltenen Pellets gezählt.

- 9 -

Die Zusammensetzung der nach den Beispielen 1 bis 6 erhaltenen Katalysatoren sowie die Ergebnisse des Siedewassertests sind in Tabelle I angegeben.

## Tabelle I

Zusammensetzung der Katalysatoren in Gew.-% und

Ergebnisse des HWT-Test

| Katalysator | $Fe_2O_3$ | $K_2O$ | MgO | $Cr_2O_3$ | $CeO_2$ | $MoO_3$ | Port- land- Zement** | Calc.Temp. | HWT |
|---|---|---|---|---|---|---|---|---|---|
| 1 (Vergleich) | 55.9 | 40.5 | – | 3.6 | – | – | – | 540°C | neg |
| 2 | 63.3 | 25.9 | 9.1 | 1.7 | – | – | – | 540°C | pos. |
| 3 (Vergleich) | 58.1 | 25.1 | – | – | 6.0 | 2.8 | 8.0 | 540°C | neg |
| 4 | 57.5 | 26.6 | 6.9 | – | 6.0 | 3.0 | – | 510°C | pos. |
| 5 (Vergleich) | 61.5 | 32.2 | 2.8 | 3.5 | – | – | – | 530°C | neg. |
| 6 | 55.3* | 14.6 | 14.0* | – | 5.9 | 2.7 | 7.5 | 550°C | pos |

*Fe und Mg vorgeformt als $MgFe_2O_4$

** Typische Zusammensetzung von Portland-Zement

| | | |
|---|---|---|
| CaO | 64.2 | Gew.-% |
| $SiO_2$ | 21.3 | " |
| $Al_2O_3$ | 5,5 | " |
| MgO | 0.9 | " |
| $Fe_2O_3$ | 2.9 | " |
| $SO_3$ | 2.4 | " |
| $TiO_2$ | 0.3 | " |
| $K_2O$ | 0.6 | " |
| $Na_2O$ | 0.3 | " |
| $B_2O_5$ | 0.3 | " |
| MnO | 0.1 | " |

- 14 -

Anwendungsbeispiel

Die in Tabelle I angegebenen Katalysatoren wurden auf
ihre Aktivität und Selektivität bei der Dehydrierung von
Ethylbenzol zu Styrol untersucht. Über den in einem Rohrreaktor mit einem Volumen von 100 cc befindlichen Katalysator
wurde ein vorgeheiztes Gemisch aus Wasserdampf und Ethylbenzol im Molverhältnis 7 : 1 geleitet. Der Katalysator
wurde dann auf derjenigen Temperatur gehalten, welche die
gewünschte Konversion des Ethylbenzols ergibt.  Der Druck
betrug 0,6 Atm, die stündliche Flüssigraumgeschwindigkeit,
bezogen auf Ethylbenzol wurde im Bereich von 0,7 bis 1,5
variiert.  Das kondensierte Produkt wurde auf Ethylbenzol,
Styrol, Benzol und Toluol analysiert,und die erhaltenen
Ergebnisse wurden auf Aktivität und Selektivität umgerechnet.

In Tabelle II bedeuten $T_{65}$ die Temperatur in °C, die notwendig ist, um ein 65%igen Umsatz von Ethylbenzol zu erhalten;
$S_{65}$ ist die bei 65%igem Umsatz erzielbare Selektivität.

### Tabelle II
Dehydrieraktivität und Selektivität bei der
Umwandlung von Ethylbenzol zu Styrol

| Katalysator | $T_{65}$ | $S_{65}$ |
|---|---|---|
| 1 | 611 | 93.6 |
| 2 | 611 | 93,6 |
| 3 | 620 | 95,3 |
| 4 | 617 | 95,0 |
| 5 | 620 | 91,0 |
| 6 | 619 | 95,5 |

Süd-Chemie AG
Lenbachplatz 6
D-8000 München 2

Europäische Patentanmeldung
4465-X-12.88
0181999

Dehydrierungskatalysator

PATENTANSPRÜCHE

1. Dehydrierungskatalysator auf der Basis von $Fe_2O_3$ und $K_2O$, dadurch erhältlich, daß man ein wasserhaltiges Gemisch aus

(a) 45 bis 90 Gew.-% $Fe_2O_3$ und/oder mindestens einer in $Fe_2O_3$ überführbaren Eisenverbindung (letztere berechnet als $Fe_2O_3$),

(b) 5 bis 40 Gew.-% $K_2O$ und/oder mindestens einer in $K_2O$ überführbaren Kaliumverbindung (letztere berechnet als $K_2O$,

(c) 4 bis 30 Gew.-% $MgO$ und/oder mindestens einer in $MgO$ überführbaren Magnesiumverbindung (letztere berechnet als $MgO$),

(d) 0 bis 10 Gew.-% einer Chrom- und/oder einer Manganverbindung, berechnet als $Me_2O_3$,

(e) 0 bis 10 Gew.-% einer Verbindung des Cers, Molybdäns, Wolframs oder deren Gemische, berechnet als das unter Normbedingungen stabilste Oxid, und

(f) 0 bis 15 Gew.-% $CaO$ und/oder mindestens einer $CaO$ enthaltenden oder in $CaO$ überführbaren Verbindung (letztere berechnet als $CaO$)

knetet und zu Formlingen verformt, die Formlinge trocknet und die getrockneten Formlinge bei Temperaturen von
500 bis 750°C calciniert.

2. Katalysator nach Anspruch 1, gekennzeichnet durch
einen Gehalt an:

50 bis 65 Gew.-% der Komponente (a)

5 bis 35 Gew.-% der Komponente (b)

5 bis 20 Gew.-% der Komponente (c)

0 bis 5 Gew.-% der Komponente (d)

0 bis 6 Gew.-% der Komponente (e)

0 bis 10 Gew.-% der Komponente (f).

3. Katalysator nach Anspruch 1, gekennzeichnet durch
einen Gehalt an

55 bis 65 Gew.-% der Komponente (a)

10 bis 30 Gew.-% der Komponente (b)

6 bis 15 Gew.-% der Komponente (c)

0 bis 3 Gew.-% der Komponente (d)

0 bis 4 Gew.-% der Komponente (e)

0 bis 5 Gew.-% der Komponente (f).

4. Katalysator nach einem der Ansprüche 1 bis 3, dadurch
erhältlich, daß man der Mischung der Komponenten (a) bis
(c) und gegebenenfalls der Komponente(n) (d) und/oder (e),
hydraulische Bindemittel, Verformungshilfsmittel und/oder die
Porosität verbessernde Mittel zusetzt.

5. Katalysator nach einem der Ansprüche 1 bis 4, dadurch
erhältlich, daß man die Formlinge bei Temperaturen von
60 bis 300°C trocknet.

6. Verwendung des Katalysators nach einem der Ansprüche
1 bis 5 zur Dehydrierung von Kohlenwasserstoffen, insbesondere von Ethylbenzol, in Gegenwart von Wasserdampf.

**Europäisches Patentamt**

# EUROPÄISCHER RECHERCHENBERICHT

## EINSCHLÄGIGE DOKUMENTE

EP 85107496.3

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| X | <u>FR - A - 2 260 380</u> (THE DOW CHEMICAL COMPANY) <br><br> * Seite 4, Zeile 13 - Seite 5, Zeile 11; Ansprüche * <br><br> -- | 1-6 | B 01 J 23/78 <br> //C 07 B 35/04 <br> C 07 C 5/333 <br> C 07 C 15/46 |
| A | <u>DE - B2 - 2 519 820</u> (INSTITUT FRANCAIS DU PETROLE) <br><br> * Tabelle 1, Kat. J; Ansprüche * <br><br> -- | 1 | |
| A | <u>US - A - 4 220 560</u> (ANQUETIL et al.) <br><br> * Ansprüche * <br><br> -- | 1 | |
| A | <u>DE - A - 2 406 280</u> (CHEMETRON) <br><br> * Seite 5, Zeile 7 - Seite 7, Zeile 6; Ansprüche * <br><br> -- | 1 | RECHERCHIERTE SACHGEBIETE (Int. Cl. 4) <br><br> B 01 J |
| A | <u>US - A - 4 467 046</u> (SMITH et al.) <br><br> * Beispiele; Ansprüche * <br><br> ---- | 1 | C 07 B <br> C 07 C |

Der vorliegende Recherchenbericht wurde für alle Patentansprüche erstellt.

| Recherchenort | Abschlußdatum der Recherche | Prüfer |
|---|---|---|
| WIEN | 26-02-1986 | TENGLER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82